(19) Europäisches Patentamt
European Patent Office
Office européen des brevets

(11) Veröffentlichungsnummer: **0 002 148**
**B1**

(12) # EUROPÄISCHE PATENTSCHRIFT

(21) Numéro de dépôt: **78400167.9**

(51) Int. Cl.³: **C 07 C 93/04**

(22) Date de dépôt: **08.11.78**

(54) **Compositions à base d'aminoalcoxyamines et procédés pour leur préparation**

(30) Priorité: **21.11.77 FR 7734846**

(43) Date de publication de la demande:
**30.05.79 Bulletin 79/11**

(45) Mention de la délivrance du brevet:
**10.12.80 Bulletin 80/25**

(84) Etats contractants désignés:
**BE CH DE GB NL**

(56) Documents cités:
**FR - A - 2 307 795**
**FR - A - 2 336 380**
**US - A - 4 036 882**

(73) Titulaire: **RHONE-POULENC INDUSTRIES**
**22, avenue Montaigne**
**F - 75008 Paris (FR)**

(72) Inventeur: **Soula, Gérard**
**33, rue Nungesser**
**F - 69330 Meyzieu (FR)**
**Leludec, Joel**
**11, rue de Boyer**
**F - 69005 Lyon (FR)**

(74) Mandataire: **Fabre, Madeleine-France, et al**
**RHONE POULENC Service Brevets Chimie et Polymères B.P. 753**
**F - 75360 Paris Cedex 08 (FR)**

**EP 0 002 148 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Compositions à base d'aminoalcoxyamines et procédés pour leur préparation

La présente invention a pour objet de nouvelles compositions à base d'aminoalcoxyamines ainsi que leur procédé de préparation.

Les demandes françaises publiées sous les n° 2.307.795 et 2.336.380 décrivent des compositions à base d'aminoalcoxyamines. Ces aminoalcoxyamines sont notamment du type:

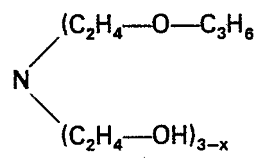

où x est un nombre entier compris entre 1 et 3, et peuvent être obtenues par cyanoéthylation de la triéthanolamine par l'acrylonitrile puis hydrogénation.

Des polymères portant des fonctions amines et présentant des motifs acétales (formales) dans leur chaine sont connus. le brevet américain n° 4.036.882 décrit par exemple ceux dérivés du bis (2-chloréthyl)formale.

Les nouvelles compositions à base d'aminoalcoxyamines objet de l'invention sont caractérisées en ce qu'elles contiennent au moins une alcoxyamine de formule:

(I) 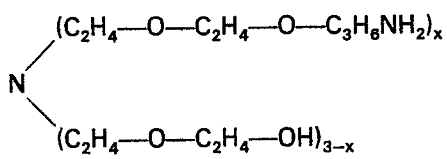

formule dans laquelle x est un nombre entier compris entre 1 et 3.

Ces dites compositions à base d'aminoalcoxyamines peuvent être préparées par cyanoéthylation de la tris(hydroxy-5 oxa-3 pentyl)amine de formule:

(II) $N-(C_2H_4-O-C_2H_4-OH)_3$

par de l'acrylonitrile selon un rapport molaire acrylonitrile/amine compris entre 1 et 4,5 suivie d'une hydrogénation des nitriles obtenus de formule:

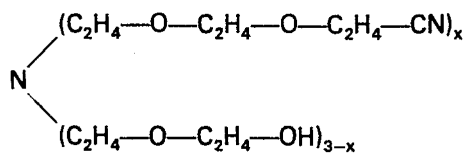

où x est un nombre entier compris entre 1 et 3.

Le rapport acrylonitrile/amine à mettre en oeuvre est fonction de la composition finale désirée.

Ainsi, lorsque le rapport molaire acrylonitrile/amine est supérieur ou égal à 3,5 on peut obtenir sélectivement la tris(amino-9 dioxa-3,6 nonyl)amine.

Lorsque ledit rapport molaire est inférieur à 3,5 on obtiendra une composition pouvant contenir les trois produits répondant à la formule (I).

La réaction de cyanoéthylation de la tris-(hydroxy-5 oxa-3 pentyl)amine peut être conduite selon les procédés généraux décrits dans la littérature (The Chemistry of Acrylonitrile, 2ème édition, Am.Cyanamid Corp.New York 1958 p.24 — H.A. BRUNSON Organic Reactions 1949, *5*, 79 — U.S. 2.326.721). Elle est effectuée habituellement en présence d'un catalyseur basique tel que le sodium, le potassium, les oxydes, hydroxydes, alcoolates ou amidures de métaux alcalins, les bases d'ammonium quaternaire telles que l'hydroxyde de triméthyl benzylammonium. La quantité d'agent alcalin à mettre en oeuvre varie de 0,1 à 5% par rapport au poids d'alcanolamine engagé. En géneral, une quantité inférieure à 1% suffit à provoquer l'effet désiré.

La réaction de cyanoéthylation est réalisée à une température comprise entre 0 et 100°C de préférence entre 30 et 50°C.

La réaction peut être conduite en présence ou non d'un solvant organique. A titre de solvants, on peut utiliser par exemple le benzène, le dioxane, la pyridine, l'acétonitrile. On peut également effecteur la réaction en présence d'eau.

Lors de l'introduction des réactifs, il est préférable de dissoudre ou disperser le catalyseur dans l'alcanolamine éventuellement diluée par le solvant et d'additionner le nitrile dans le milieu sous agitation.

Le produit de cyanoéthylation obtenu précédemment peut être hydrogéné directement ou éventuellement après isolement par tout moyen approprié. On peut opérer sa réduction par les procédés usuels de réduction des nitriles — HOUBEN-WEYL — methoden der Organischen Chemie — 4ème édition — Band XI/559 (1957). La voie la plus couramment utilisée est l'hydrogénation en présence de catalyseurs au nickel ou cobalt déposés ou non sur support. Mais on utilise plus particulièrement le nickel Raney ou le cobalt Raney mis en jeu dans une proportion de 5 à 30% par rapport au poids du nitrile traité. Il est avantageux de réaliser l'hydrogénation avec de tels catalyseurs dans l'ammoniac liquide ou de préférence en présence d'une base en milieu aqueux ou organique. Comme agent basique, on peut utiliser l'hydroxyde de baryum, de sodium, de potassium ou de lithium ou les hydroxydes d'ammonium quaternaire. La quantité de base mise en oeuvre exprimée par rapport au poids de nitrile traité est d'environ 1 à 30%.

L'hydrogénation peut se dérouler dans un solvant organique inerte dans les conditions de

la réaction. On peut faire appel à des alcools aliphatiques inférieurs comme le méthanol, l'éthanol, le propanol, l'isopropanol; à des diols comme l'éthanediol-1,2, le propanediol-1,2; à des éthers-oxydes comme l'éther éthylique, l'éther butylique, le diméthoxyéthane, le tétrahydrofuranne, le dioxanne; à des éthers-oxydes partiels de composés polyhydroxylés comme l'éther monométhylique (ou monoéthylique) de l'éthylene-glycol.

La réaction peut être réalisée à une température comprise entre 30 et 100°C de préférence entre 60 et 80°C et sous une pression d'hydrogène de 10 à 200 bars; en général, une pression de 20 à 30 bars convient bien pour effectuer l'hydrogénation.

En pratique, le produit de cyanoéthylation est additionné progressivement dans la suspension du catalyseur maintenu sous la pression d'hydrogène choisie.

Après la réaction d'hydrogénation, le catalyseur est séparé, l'agent basique est neutralisé, le solvant est éliminé et les aminoalcoxyamines peuvent être distillées sous pression réduite.

La tris(hydroxy-5 oxa-3 pentyl)amine de formule II mise en oeuvre pour préparer les aminoalcoxyamines objet de l'invention peut être préparée selon le procédé décrit dans le brevet anglais n° 364,000 par action de l'oxyde d'éthylène sur une solution d'ammonlaque à 20%. Il est toutefois préféable de préparer ce produit de formule II par condensation d'un monoglycolate de métal alcalin (sodium ou potassium) en solution dans du glycol sur du chlorhydrate de tris(chloro-2 éthyl)amine selon un rapport molaire monoglycolate/chlorhydrate ⩾4; l'opération de condensation est réalisée à la température de reflux du glycol (197°C) pendant 2 à 6 heures, à l'aide d'une solution glcyolique contenant de 0,5 à 5 moles de monoglycolate de métal alcalin, de préférence de 2 à 4 moles, par litre de glycol.

Les compositions, objet de l'invention, peuvent être utilisées comme intermédiaires de fabrication d'additifs pour huiles lubrifiantes et comme catalyseurs ou agents réticulants dans la synthèse des polyuréthannes.

Les exemples suivants sont donnés à titre indicatif et ne peuvent être considérés comme une limite du domaine et de l'esprit de l'invention.

## EXEMPLE 1

*Cyanoéthylation*

Dans un ballon de 250 ml muni d'un agitateur mecanique, d'un thermomètre, d'un réfrigérant et d'une ampoule à brome, on introduit 70,5 g de tris(hydroxy-5 oxa-3 pentyl)-amine (0,25 mole), 0,46 ml de soude à 36°Bé (4,6 . $10^{-3}$ mole). Le mélange est chauffé à 38°C et on introduit alors 53 g d'acrylonitrile (1 mole) en 1 heure.

En fin d'addition d'acrylonitrile, on ajoute 0,6 ml d'HCl (d = 1,19) pour neutraliser la soude. Le chlorure de sodium précipite. Après filtration, on obtient une huile de couleur jaune paille, constituée de tris(cyano-8 dioxa-3,6 octyl)-amine.

*Hydrogénation*

Ce produit est hydrogéné dans un autoclave sous une pression d'hydrogène de 40 bars à 60°C, en présence de 60 g de nickel Raney en suspension dans 130 ml d'éthanol et de 0,75 ml de soude. La réaction s'effectue en 3 heures. Après refroidissement, l'autoclave est dégazé et ouvert sous azote. on filtre, Après élimination du catalyseur on obtient 110 g d'huile, ce qui correspond à un rendement de 97%.

La micro-analyse élémentaire confirme que le produit obtenu est de la tris(amino-9 dioxa-3,6 nonyl) amine.

| | valeur trouvée % | valeur calculée% |
|---|---|---|
| Carbone | 55,32 | 55,75 |
| Hydrogène | 10,68 | 10,62 |
| Azote | 12,5 | 12,39 |
| Oxygène | 21,5 | 21,24 |

Le produit est obtenu avec 98% de pureté.

## EXEMPLE 2

*Cyanoéthylation*

Dans un ballon de 250 ml muni d'un agitateur mécanique, d'un thermomètre, d'un réfrigérant et d'une ampoule à brome, on introduit 70,5 g de tris(hydroxy-5 oxa-3 pentyl)-amine (0,25 mole), 0,46 ml de soude à 36°Bé (4,6 . $10^{-3}$ mole). Le mélange est chauffé à 38°C et on introduit alors 42 g d'acrylonitrile (0,79 mole) en 1 heure. En fin d'addition d'acrylonitrile, on ajoute 0,6 ml d'HCl (d = 1,19) pour neutraliser la soude. Le chlorure de sodium précipite. Après filtration on obtient une huile de couleur jaune paille.

*Hydrogénation*

Dans un autoclave en inox de 1 litre muni d'un système d'agitation, équipé d'une réserve d'hydrogène de 250 ml, on introduit 110,3 g de produit brut précédemment fabriqué en solution dans 110 ml d'éthanol absolu, puis on ajoute 56,9 g de nickel Raney en suspension dans 130 ml d'éthanol et 0,75 ml de soude (36°Bé soit 4,8 . $10^{-3}$ mole).

L'autoclave est fermé et purgé par 3 fois à l'azote (pression 10 bars) puis l'hydrogène est introduit. On monte la pression à 40 bars à température ambiante à l'aide du mano-détenteur commandant l'arrivée d'hydrogène de la réserve. Le mélange est chauffé à 60°C.

L'absorption d'hydrogène cesse au bout de 2h 30. Après refroidissement, l'autoclave est dégazé et ouvert sous azote.

On filtre, lave le catalyseur à l'éthanol. Après élimination de l'éthanol, on obtient une huile jaune.

On obtient 105,4 g de produit, ce qui correspond à un rendement de 93%.

Le produit est constitué de:

— 85% de tris(amino-9 dioxa-3,6 nonyl)-amine

— 12% de bis NN(amino-9-dioxa-3,6 nonyl)-N(hydroxy-5 oxa-3 pentyl)amine

— 3% de bis NN(hydroxy-5 oxa-3 pentyl)N-(amino-9 dioxa-3,6 nonyl)amine

EXEMPLE 3

*Cyanoéthylation*

Avec le même appareillage que celui décrit dans les exemples 1 et 2, on effectue la réaction suivante:

A 70,5 g (0,25 mole) de tris(hydroxy-5 oxa-3 pentyl)amine on ajoute 0,46 ml de soude 36°Bé. Le mélange est chauffé à 38°C et on introduit alors 20 g d'acrylonitrile (0,375 mole) en 1 h. En fin d'addition d'acrylonitrile on ajoute 0,6 ml d'HCl (d = 1,19) pour neutraliser la soude. Le chlorure de sodium précipite. Après filtration, on obtient une huile de couleur jaune paille dont le composition est la suivante:

| | |
|---|---|
| Triol résiduel | 10% |
| Triol dicyanoéthylé | 40% |
| Triol monocyanoéthylé | 40% |
| Triol tricyanoéthylé | 10% |

*Hydrogénation*

Ce mélange est hydrogéné selon le mode opératoire décrit dans l'exemple précédent. Le mélange est chauffé à 60°C. L'absorption d'hydrogène cesse au bout de 1 h 30. Après refroidissement, l'autoclave est dégazé et ouvert sous azote. On filtre et lave le catalyseur à l'éthanol. Après élimination de l'éthanol, on effectue une distillation flash à 210°C sous 0,5 mm de Hg pour récupérer en tête le triol non transformé et en pieds le mélange d'aminoalcoxyamines qui se compose de:

— 44% de NN bis (hydroxy-5 oxa-3 pentyl)-N(amino-9 dioxa-3,6 nonyl)amine

— 44% de N(hydroxy-5 oxa-3 pentyl)NN bis-(amino-9 dioxa-3,6 nonyl)amine

— 12% de NNN tris(amino-9 dioxa-3,6 nonyl)amine.

EXEMPLE 4

Avec le même appareillage que celui décrit dans l'exemple 1 et l'exemple 2, on effectue la réaction suivante:

A 70,5 g (0,25 mole) de tris(hydroxy-5 oxa-3 pentyl)amine on ajoute 0,46 ml de soude 36°Bé ($4,6. 10^{-3}$ mole). Le mélange est chauffé à 38°C et on introduit alors 24,6 g d'acrylonitrile (0,465 mole) en 2 heures. En fin d'addition d'acrylonitrile on ajoute 0,6 ml d'HCl (d = 1,19) pour neutraliser la soude. Le chlorure de sodium précipite. Après filtration, on obtient une huile jaune qui a la composition suivante:

| | |
|---|---|
| — Triol résiduel | 4 % |
| — Triol monocyanoéthylé | 22% |
| — Triol dicyanoéthylé | 58% |
| — Triol tricyanoéthylé | 16% |

Ce mélange est hydrogéné selon le mode opératoire décrit dans l'exemple 1. On chauffe à 60°C. Dans ces conditions, l'absorption d'hydrogène cesse au bout de 2 heures. Après refroidissement, on dégaze l'autoclave et on l'ouvre sous azote. On filtre, lave le catalyseur à l'éthanol. Après élimination de l'éthanol, on effectue une distillation flash à 210°C sous une pression de 0,5 mm de Hg, pour récupérer en tête le triol non transformé et en pieds le mélange de polyamines. Ce mélange est analysé par chromatographie en phase gazeuse. Il se compose de:

— 23% de NN bis (hydroxy-5 oxa-3 pentyl)-N(amino-9 dioxa-3,6 nonyl)amine

— 60% de N(hydroxy-5 oxa-3 pentyl)NN bis-(amino-9 dioxa-3,6 nonyl)amine

— 17% de NNN tris(amino-9 dioxa-3,6 nonyl)amine.

La tris(oxa-3 hydroxy-5 pentyl)amine utilisée aux exemples précédents pour préparer les aminoalcoxyamines, est préparée de la manière suivante:

*— Préparation de glycolate de sodium*

Dans un ballon de 3 litres, muni d'un agitateur mécanique d'un thermomètre, d'une tête de distillation, suivie d'un réfrigérant et d'un ballon récepteur, on introduit 2200 g (soit 35,5 mole) de glycol, 218,5 g (5,35 moles) de soude en pastilles (à 98%).

Vers 85°C la soude est totalement dissoute. On recueille la majorité de l'eau (80 ml) en chauffant jusqu'à 125°C, et le reste distille avec le glycol de 125° à 135°C. La solution ainsi obtenue est refroidie jusqu'à 80°C.

*— Condensation avec le chlorhydrate de tris-(chloro-2 éthyl)amine*

A cette solution de glycolate de sodium dans le glycol, on ajoute 280,4 g (soit 1,16 mole) de chlorhydrate de tris(chloro-2 éthyl)amine qui peut être préparé à partir du chlorhydrate de triéthanolamine selon la méthode décrite par exemple par K. WARD — JACS 57, 914—1953 —); on chauffe au reflux à 197°C pendant 2 heures On observe la précipitation de chlorure de sodium.

Une grande partie du glycol (1450 ml) est ensuite distillée sous 3 mm Hg pendant 4 h 30.

Le contenue du ballon est alors filtré; le gateau est lavé par 6 fois 100 ml d'acétone, puis séché.

On a récupéré 270 g de chlorure de sodium contre 272 g selon la théorie.

Le glycolate en excès est neutralisé avec 0,7 mole (soit 56,5 ml) d'acide chlorhydrique (d = 1,19). L'acétone est évaporée, et le résidu huileux obtenu est distillé. On recueille le coeur de distillation (entre 210—220°C) sous 2 mm Hg. On récupère ainsi 213 g de liquide jaune clair, constitué de tris(hydroxy-5 oxa-3 pentyl)-amine. La nature de ce produit a pu être confirmée par analyse infra-rouge, micro-analyse et par résonnance magnétique nucléaire.

**Revendications**

1. Compositions à base d'aminoalcoxyamines caractérisées en ce qu'elles contiennent au moins une aminoalcoxyamine de formule:

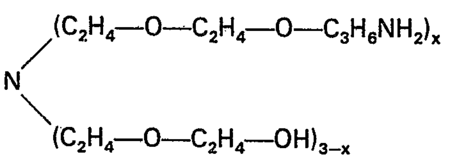

formule dans laquelle x est un nombre entier compris entre 1 et 3.

2. Composition selon la revendication 1 caractérisée en ce qu'elle est constituée de tris-(amino-9 dioxa-3,6 nonyl)amine.

3. Composition selon la revendication 1 caractérisée en ce qu'elle est constituée d'environ 85% de tris(amino-9 dioxa-3,6 nonyl)-amine, 12% de bis NN(amino-9 dioxa-3,6 nonyl)N(hydroxy-5 oxa-3 pentyl)amine et 3% de bis NN(hydroxy-5 oxa-3 pentyl)N(amino-9 dioxa-3,6 nonyl)amine.

4. Composition selon la revendication 1 caractérisée en ce qu'elle est constituée d'environ 60% de N(hydroxy-5 oxa-3 pentyl)-amine NNbis (amino-9 dioxa-3,6 nonyl)amine, 23% de NNbis (hydroxy-5 oxa-3 pentyl)N-(amino-9 dioxa-3,6 nonyl)amine et 17% de NNN tris (amino-9 dioxa-3,6 nonyl)amine.

5. Composition selon la revendication 1 caractérisée en ce qu'elle est constituée d'environ 44% de N,N bis (hydroxy-5 oxa-3 pentyl) N(amino-9 dioxa-3,6 nonyl)amine, 44% de N(hydroxy-5 oxa-3 pentyl)NN bis (amino-9 dioxa-3,6 nonyl)amine et 12% de NNN tris-(amino-9 dioxa-3,6 nonyl)amine.

6. Procédé de préparation des compositions faisant l'objet de la revendication 1 caractérisé en ce que l'on réalisé une cyanoéthylation de la tris(hydroxy-5 oxa-3 pentyl)amine par de l'acrylonitrile selon un rapport molaire acrylonitrile/amine compris entre 1 et 4,5 puis une hydrogénation du ou des nitriles obtenus.

7. Procédé de préparation de la composition faisant l'objet de la revendication 2 caractérisé en ce que l'on réalise une cyanoéthylation de la tris(hydroxy-5 oxa-3 pentyl)amine par de l'acrylonitrile selon un rapport molaire acrylonitrile/amine au moins égal à 3,5 puis une hydrogénation de la tris(cyano-8 dioxa-3 octyl)-amine obtenue.

8. Procédé de préparation de la composition faisant l'objet de la revendication 3, caractérisé en ce que l'on réalise une cyanoéthylation de la tris(hydroxy-5 oxa-3 pentyl)amine par de l'acrylonitrile selon un rapport molaire acrylonitrile/amine de l'ordre de 3,1 puis une hydrogénation des nitriles obtenus.

9. Procédé de préparation de la composition faisant l'objet de la revendication 4 caractérisé en ce que l'on réalise une cyanoéthylation de la tris(hydroxy-5 oxa-3 pentyl)amine par de l'acrylonitrile selon un rapport molaire acrylonitrile/amine de l'ordre de 1,8 puis une hydrogénation des nitriles obtenus.

**Patentansprüche**

1. Zubereitungen auf der Basis von Aminoalkoxyaminen, dadurch gekennzeichnet, daß sie mindestens ein Aminoalkoxyamin der Formel

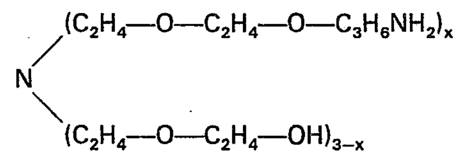

enthalten, in der x eine ganze Zahl von 1 bis 3 ist.

2. Zubereitung nach Anspruch 1, dadurch gekennzeichnet, daß sie aus Tris(9-amino-3,5-dioxanonyl)amin besteht.

3. Zubereitung nach Anspruch 1, dadurch gekennzeichnet, daß sie aus etwa 85% Tris(9-amino-3,6-dioxanonyl)amin, 12% Bis-N,N(9-amino - 3,6 - dioxanonyl) - N - (5 - hydroxy - 3 - oxapentyl)amin und 3% Bis - N,N - (5 - hydroxy - 3 - oxapentyl) - N - (9 - amino - 3,6 - dioxa - nonyl)amin besteht.

4. Zubereitung nach Anspruch 1, dadurch gekennzeichnet, daß sie aus etwa 60% N(5-Hydroxy - 3 - oxapentyl)amin - N,N - bis(9 - amino - 3,6 - dioxanonyl)amin, 23% N,N - Bis - (5 - hydroxy - 3 - oxapentyl) - N - (9 - amino - 3,6 - dioxanonyl)amin und 17% N,N,N - Tris - (9 - amino - 3,6 - dioxanonyl)amin besteht.

5. Zubereitung nach Anspruch 1, dadurch gekennzeichnet, daß sie aus etwa 44% N,N - Bis - (5 - hydroxy - 3 - oxapentyl) - N - (9 - amino - 3,6 - dioxanonyl)amin, 44% N(5 - Hydroxy - 3 - oxapentyl) - N,N - bis(9 - amino - 3,6 - dioxa - nonyl)amin und 12% N,N,N - Tris(9 - amino - 3,6 - dioxanonyl)amin besteht.

6. Verfahren zur Herstellung der Zubereitungen nach Anspruch 1, dadurch gekennzeichnet, daß man Tris(5-hydroxy-3-oxapentyl)amin mit Acrylnitril in einem Molverhältnis Acrylnitril/Amin von 1 bis 4,5 cyan-äthyliert und das oder die erhaltenen Nitrile hydriert.

7. Verfahren zur Herstellung der Zuberietung

nach Anspruch 2, dadurch gekennzeichnet, daß man Tris(5-hydroxy-3-oxapentyl)amin mit Acrylnitril in einem Molverhältnis Acrylnitril/Amin von mindestens 3,5 cyan-äthyliert und dann das erhaltene Tris(8 - cyano - 3,6 - dioxaoctyl)amin hydriert.

8. Verfahren zur Herstellung der Zubereitung nach Anspruch 3, dadurch gekennzeichnet, daß man Tris(5 - hydroxy - 3 - oxapentyl)amin mit Acrylnitril in einem Molverhältnis Acrylnitril/Amin von 3,1 cyanäthyliert und dann die erhaltenen Nitrile hydriert.

9. Verfahren zur Herstellung der Zubereitung nach Anspruch 4, dadurch gekennzeichnet, daß man Tris(5-hydroxy-3-oxapentyl)amin mit Acrylnitril in einem Molverhältnis Acrylnitril/Amin von 1,8 cyanäthyliert und die erhaltenen Nitrile hydriert.

## Claims

1. Compositions based on aminoalkoxyamines, characterised in that they contain at least one aminoalkoxyamine of the formula:

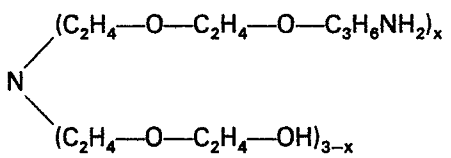

in which formula x is an integer between 1 and 3.

2. Composition according to Claim 1, characterised in that it consists of tris-(9-amino-3,6-dioxanonyl)amine.

3. Composition according to Claim 1, characterised in that it consists of about 85% of tris-(9 - amino - 3,6 - dioxanonyl) - amine, 12% of N,N - bis - (9 - amino - 3,6 - dioxanonyl) - N - (5 - hydroxy - 3 - oxapentyl) - amine and 3% of N,N - bis - (5 - hydroxy - 3 - oxapentyl) - N - (9 - amino - 3,6 - dioxanonyl)amine.

4. Composition according to Claim 1, characterised in that it consists of about 60% of N - (5 - hydroxy - 3 - oxapentyl) - amine - N,N - bis - (9 - amino - 3,6 - dioxanonyl) - amine, 23% of N,N - bis - (5 - hydroxy - 3 - oxapentyl) - N - (9 - amino - 3,6 - dioxanonyl) - amine and 17% of N,N,N - tris - (9 - amino - 3,6 - dioxanonyl) - amine.

5. Composition according to Claim 1, characterised in that it consists of about 44% of N,N - bis - (5 - hydroxy - 3 - oxapentyl) - N - (9 - amino - 3,6 - dioxanonyl) - amine, 44% of N - (5 - hydroxy - 3 - oxapentyl) - N,N - bis - (9 - amino - 3,6 - dioxanonyl) - amine and 12% of N,N,N - tris - (9 - amino - 3,6 - dioxanonyl) - amine.

6. Process for the preparation of the compositions forming the subject of Claim 1, characterised in that tris-(5-hydroxy-3-oxapentyl)-amine is cyanoethylated with acrylonitrile in a molar ratio acrylonitrile/amine of between 1 and 4.5, and the nitrile or nitriles obtained are then hydrogenated.

7. Process for the preparation of the composition forming the subject of Claim 2, characterised in that tris-(5-hydroxy-3-oxapentyl)-amine is cyanoethylated with acrylonitrile in a molar ratio acrylonitrile/amine equal to at least 3.5, and the tris-(8-cyano-3,6-dioxaoctyl)-amine obtained is then hydrogenated.

8. Process for the preparation of the composition forming the subject of Claim 3, characterised in that tris-(5-hydroxy-3-oxapentyl)-amine is cyanoethylated with acrylonitrile in a molar ratio acrylonitrile/amine of the order of 3.1, and the nitriles obtained are then hydrogenated.

9. Process for the preparation of the composition forming the subject of Claim 4, characterised in that tris-(5-hydroxy-3-oxapentyl)-amine is cyanoethylated with acrylonitrile in a molar ratio acrylonitrile/amine of the order of 1.8, and the nitriles obtained are then hydrogenated.